# EUROPEAN PATENT APPLICATION

(11) **EP 2 636 419 A1**
(43) Date of publication of application: **11.09.2013**
(21) Application number: 12158497.3
(22) Date of filing: 07.03.2012
(51) Int. Cl.: A61M 5/32

(54) **Needle assembly removal device and disposal device**

(71) Applicant: Sanofi-Aventis Deutschland GmbH, 65929 Frankfurt am Main (DE)
(72) Inventor: The designation of the inventor has not yet been filed

(57) **Abstract**

Described is a needle assembly removal device (100) comprising a coupling mechanism adapted to engage a disposal device, a housing (105), and a carriage (130) adapted to engage a needle assembly (400). Axial movement of the carriage (130) relative to the housing (105) causes the needle assembly (400) to disengage a medicament delivery device (300). Also described is a disposal device (200) comprising an interface (205) adapted to engage the needle assembly removal device (100) and a resilient seal (210) covering the interface (205).

## Description

### Technical Field

The invention relates to a device for removing a needle assembly from a medicament delivery device and a disposal device for safely discarding the removed needle assembly.

### Background of the Invention

Administering a medicament by injection is a process which presents a number of risks and challenges for patients and healthcare professionals, both mental and physical. Improper handling of medicament delivery devices, before, during and after an injection, may result in needle stick injuries. In any situation in which a patient or healthcare professional is required to manually remove the needle assembly, there is a risk of needle stick injury.

Conventional needle assemblies are attached to medicament delivery devices by a threaded engagement. Thus, after an injection, either a patient or a physician/nurse will have to remove the needle assembly by unscrewing it. As long as the needle assembly must be touched, there exists the risk of a needle stick injury.

Thus, there is a need for a device for safely removing a needle assembly from a medicament delivery device and a disposal device for discarding the removed needle assembly.

### Summary of the Invention

It is an object of the present invention to provide a device for removing a needle assembly from a medicament delivery device and a disposal device for safely discarding the removed needle assembly.

In an exemplary embodiment, a needle assembly removal device according to the present invention comprises a coupling mechanism adapted to engage a disposal device, a housing, and a carriage adapted to engage a needle assembly. Axial movement of the carriage relative to the housing causes the needle assembly to disengage a medicament delivery device.

In an exemplary embodiment, the coupling mechanism includes at least one of threads, a bayonet mechanism, a friction fit and a snap fit.

In an exemplary embodiment, the coupling mechanism is adapted to detachably engage the disposal device.

In an exemplary embodiment, the housing includes a proximal opening adapted to receive the medicament delivery device and a base with a distal opening.

In an exemplary embodiment, the housing includes a track adapted to engage the carriage. The track includes one or more helical grooves. The carriage includes at least one peg adapted to engage the one or more helical grooves.

In an exemplary embodiment, the carriage is adapted to rotate in a first rotational direction when the carriage moves in a first axial direction relative to the housing and the carriage is adapted to rotate in a second rotational direction when the carriage moves in a second axial direction relative to the housing.

In an exemplary embodiment, the needle assembly removal device further comprises a spring adapted to apply a biasing force to the carriage. The spring biases the carriage in the second axial direction.

In an exemplary embodiment, the carriage includes a retaining portion adapted to engage a needle hub of the needle assembly and prevent the needle assembly from rotating relative to the carriage. The carriage includes a slot separating the retaining portion. The retaining portion is adapted to deflect radially via the slot to accommodate the needle hub. The retaining portion includes a ramped portion adapted to engage a stem on the base. The retaining portion deflects radially when the ramped portion engages the stem.

In an exemplary embodiment, a disposal device according to the present invention comprises an interface adapted to engage the needle assembly removal device and a resilient seal covering the interface. The disposal device may further comprise a cap adapted to cover the interface and the seal.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
Figure 1 shows an exemplary embodiment of a needle assembly removal device and a disposal device according to the present invention before use,
Figures 2A and 2B show an exemplary embodiment of a needle assembly removal device according to the present invention,
Figure 3 shows an exemplary embodiment of a needle assembly removal device and a disposal device according to the present invention during use,
Figure 4 shows an exemplary embodiment of a needle assembly removal device according to the present invention during use,
Figure 5 shows an exemplary embodiment of a needle assembly removal device and a disposal device according to the present invention during use,
Figure 6 shows an exemplary embodiment of a needle assembly removal device according to the present invention during use,
Figure 7 shows an exemplary embodiment of a needle assembly removal device according to the present invention during use,
Figure 8 shows an exemplary embodiment of a needle assembly removal device and a disposal device according to the present invention during use,
Figure 9 shows an exemplary embodiment of a needle assembly removal device according to the present invention during use,
Figure 10 shows an exemplary embodiment of a needle assembly removal device and a disposal device according to the present invention after use, and
Figure 11 shows an exemplary embodiment of a needle assembly removal device and a disposal device according to the present invention after use.

Corresponding parts are marked with the same reference symbols in all figures.

### Detailed Description

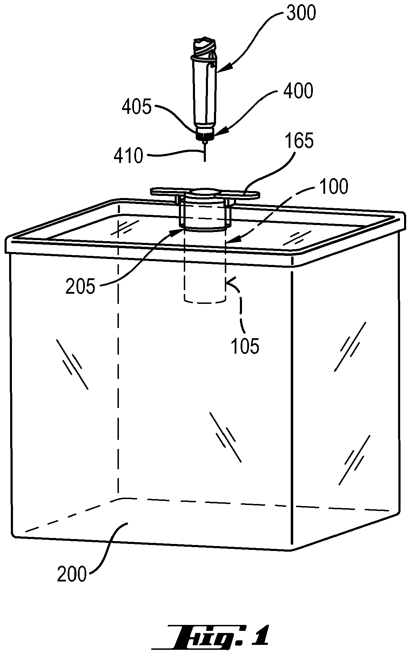

Figure 1 shows an exemplary embodiment of a needle assembly removal device 100 and a disposal device 200 according to the present invention. Figure 1 also shows a medicament delivery device 300 having a needle assembly 400 detachably coupled thereto. The medicament delivery device 300 may be a pen injector, an auto-injector, a syringe, a perfusion/infusion system, etc. The medicament delivery device 300 may contain a syringe, cartridge or ampoule filled with a medicament, or the medicament delivery device 300 may be in fluid communication with a medicament source.

In an exemplary embodiment, the needle assembly 400 includes a needle hub 405 which engages the medicament delivery device 300, and a needle 410 coupled to the needle hub 405. The needle 410 may be a double-tipped needle having a proximal tip adapted to pierce a septum on cartridge/ampoule and a distal tip adapted to pierce an injection site. In an exemplary embodiment, the needle hub 405 includes one or more threads adapted to engage a corresponding thread on the medicament delivery device 300. In other exemplary embodiments, the needle hub 405 may engage the medicament delivery device 300 via another coupling mechanism such as, for example, a bayonet mechanism, a friction fit, a snap fit, etc.

The disposal device 200 may be a sharps disposal unit which is adapted to contain used needle assemblies in a safe manner.

In an exemplary embodiment, the needle assembly removal device 100 is removably coupled to the disposal device 200. For example, the disposal device 200 may have an interface 205 (e.g., a hole) which is adapted to receive the needle assembly removal device 100 and provide access to an interior of the disposal device 200. In an exemplary embodiment, the interface 205 may have threads (or another coupling mechanism) for engaging corresponding threads on the needle assembly removal device 100. Thus, in an exemplary embodiment, the needle assembly removal device 100 may be portable. In another exemplary embodiment, the needle assembly removal device 100 may be formed integrally with the disposal device 200.

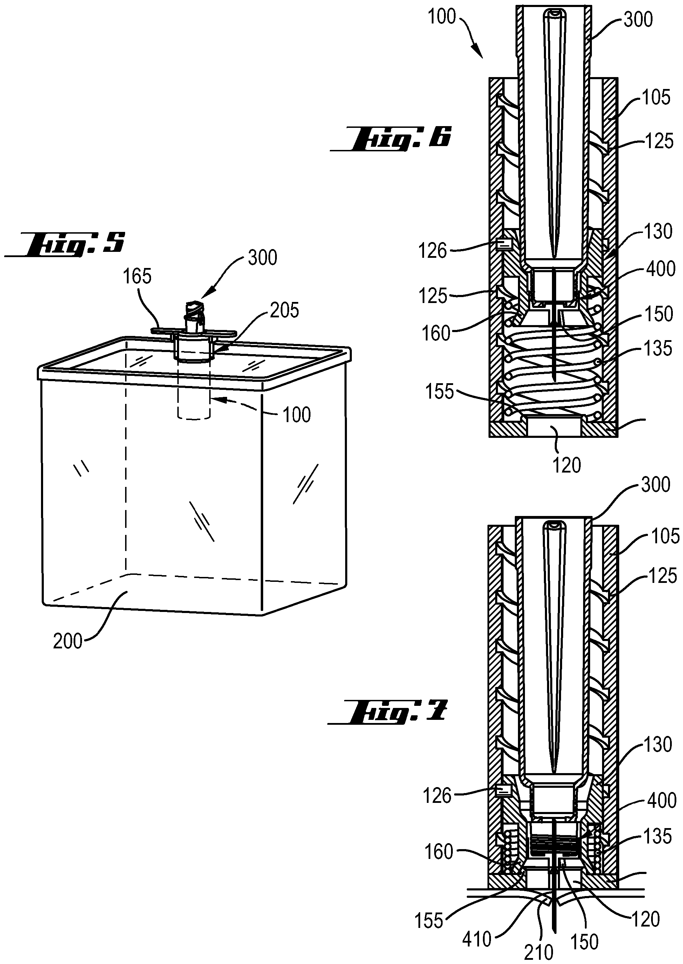

In an exemplary embodiment, the interface 205 includes a resilient seal 210 (shown in Figure 7) adapted to deflect and allow insertion of a used needle assembly into the disposal device but return to a sealed configuration after insertion. The seal 210 may prevent release of pathogens, microbes, etc. after the needle assembly removal device 100 is removed from the disposal device 200 and/or when the needle assembly removal device 100 is not in use.

While the exemplary embodiment show in Figure 1 depicts the needle assembly removal device 100 passing through the interface 205 and extending, at least partially, into the interior of the disposal device 200, those of skill in the art will understand that a distal opening of the needle assembly removal device 100 may be substantially coplanar with the seal 210. Thus, in another exemplary embodiment, the needle assembly removal device 100 may not have any portion which extends into the interior of the disposal device 200.

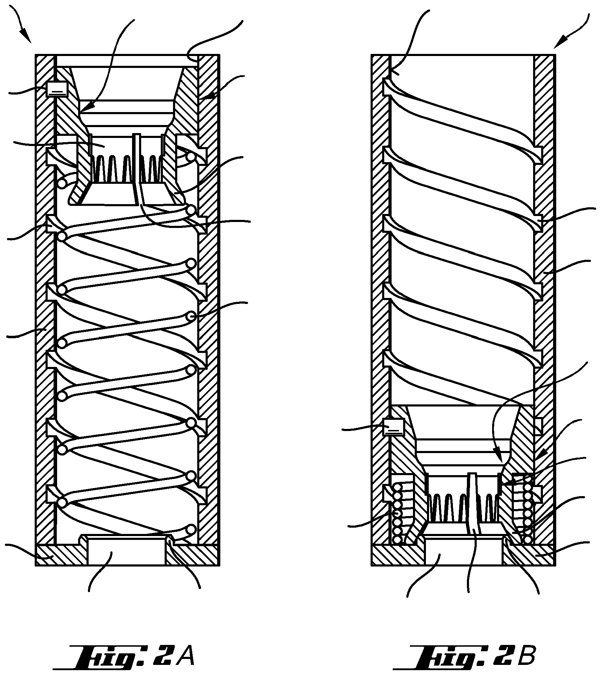

Figures 2A and 2B show an exemplary embodiment of a needle assembly removal device 100 according to the present invention. The needle assembly removal device 100 includes a housing 105, which may be cylindrical and have a proximal opening 110. A base 115 having a distal opening 120 may be formed on or coupled to a distal end of the housing 105. At least a portion of an external surface of the housing 105 may have a coupling mechanism (not shown), e.g., threads, formed thereon for engaging the corresponding coupling mechanism on the interface 205 of the disposal device 200.

A track 125 may be formed on an internal surface of the housing 105. In an exemplary embodiment, the track 125 is one or more helical grooves which extend from a proximal end of the housing 105 to the distal end.

A carriage 130 disposed in the housing 105 engages the track 125. For example, the carriage 130 may include one or more pegs 126 adapted to engage the one or more helical grooves. In the exemplary embodiment, the carriage 130 rotates in a first rotational direction when moving distally relative to the housing 105 and rotates in a second rotational direction opposite the first rotational direction when moving proximally relative to the housing 105. A spring 135 may bias the carriage 130 towards the proximal end of the housing 105.

In an exemplary embodiment, the carriage 130 is adapted to engage the needle assembly 400. For example, the carriage 130 may include a proximal guide portion 140 which is adapted to receive the needle assembly 400 and align it with a retaining portion 145. The retaining portion 145 may include a contoured surface (e.g., grooves, channels, ribs, etc.) or a frictional surface to engage the needle hub 405 and prevent rotation of the needle hub 405 relative to the carriage 130.

In an exemplary embodiment, a diameter of the retaining portion 145 may be substantially equal to or less than a diameter of the needle hub 405. The carriage 130 may include a slot 150 formed in at least a portion of the retaining portion 145. As described further below, when the needle assembly 400 is inserted into the carriage 130, the needle hub 405 may cause the retaining portion 145 to deflect via the slot 150. The retaining portion 145 may frictionally engage the needle hub 405 to prevent the needle assembly 400 from rotating relative to the carriage 130.

In an exemplary embodiment, the base 115 includes a stem 155 formed adjacent to the distal opening 120. As explained further below and shown in Figure 2B, the stem 155 engages a ramped portion 160 of the retaining portion 145 to deflect the retaining portion 145 via the slot 150, which causes the retaining portion 145 to disengage the needle assembly 400. The needle assembly 400 then passes through the interface 205 and into the interior the disposal device 200.

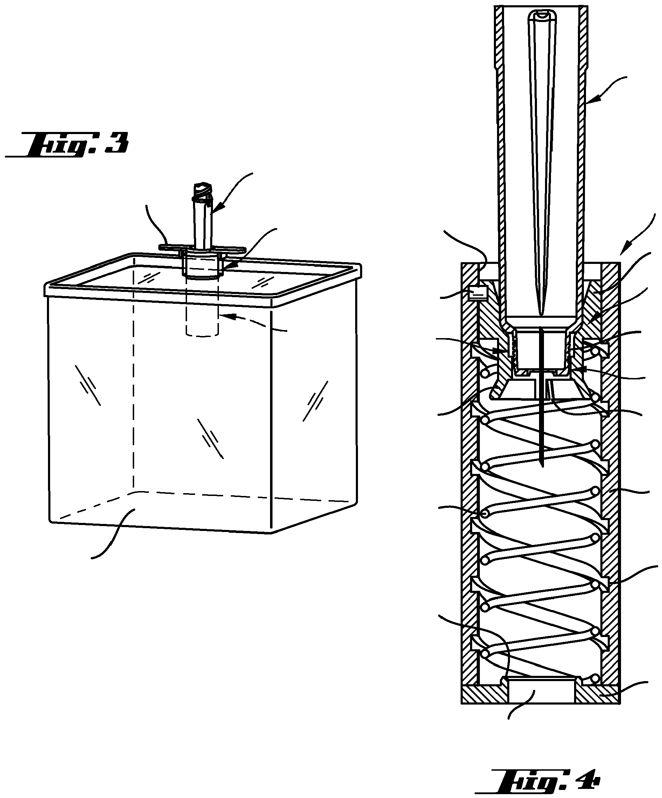

Figures 3 and 4 show an exemplary embodiment of a needle assembly removal device 100 and a disposal device 200 according to the present invention during use. In an exemplary embodiment, the needle assembly removal device 100 may have one or more handles 165 which facilitate securing the needle assembly removal device 100 to the disposal device 200. For example, the handle 165 may be used to rotate the needle assembly removal device 100 relative to the disposal device 200 to engage corresponding threaded surfaces.

As shown in Figure 4, the carriage 130 is in a first axial position (e.g., a proximal position) relative to the housing 105. When the medicament delivery device 300 is inserted into the carriage 130, the distal end of the medicament delivery device 300 may be aligned by the guide portion 140 which directs the needle assembly 400 into the retaining portion 145. When the needle hub 405 engages the retaining portion 145, the retaining portion 145 may frictionally engage the needle hub 405 and/or engage a contoured surface of the needle hub 405. For example, the retaining portion 145 may have ribs which engage grooves on the needle hub 405, or vice-versa, and/or the retaining portion 145 may deflect via the slot 150, expanding to accommodate the needle hub 405. The retaining portion 145 secures the needle hub 405 and prevents the needle assembly 400 from rotating relative to the carriage 130.

In an exemplary embodiment, a force required to compress the spring 135 is greater than a force required to deflect the retaining portion 145. Thus, the spring 135 may prevent the carriage 130 from translating relative to the housing 105 until the needle assembly 400 is secured in the carriage 130.

Figures 5, 6 and 7 show an exemplary embodiment of a needle assembly removal device 100 and a disposal device 200 according to the present invention during use. As the medicament delivery device 300 is inserted into the needle assembly removal device 100, the needle assembly 400 is disengage from the medicament delivery device 300. For example, in the exemplary embodiment shown in Figure 6, as the carriage 130 moves axially relative to the housing 105 in the distal direction, the carriage 130 rotates relative to the housing 105, because the pegs 126 on the carriage 130 follow the track 125. Because the needle assembly 400 does not rotate relative to the carriage 130, rotation of the carriage 130 causes rotation of the needle assembly 400 relative to the medicament delivery device 300, disengaging the threaded connection therebetween.

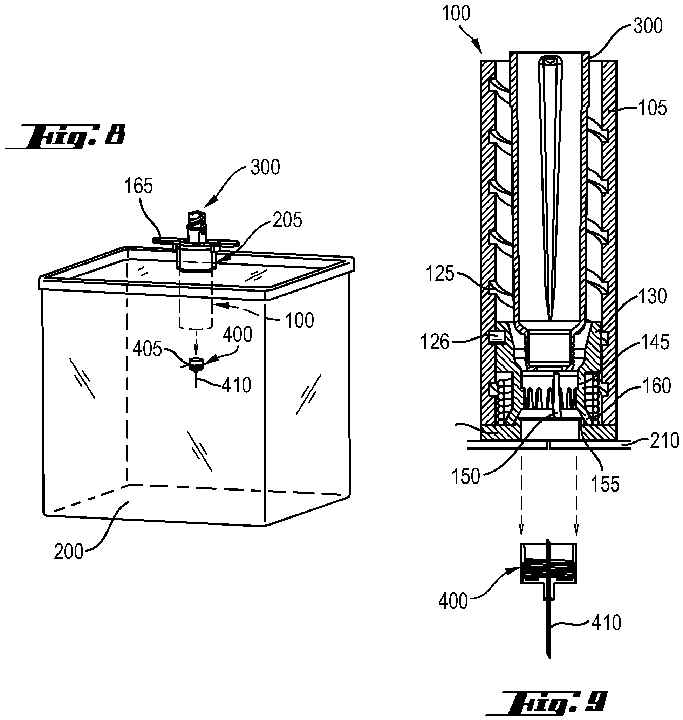

As shown in Figure 7, when the carriage 130 reaches a second axial position (e.g., a distal position) relative to the housing 105, the needle 410 deflects the seal 210. Also, the ramped portion 160 of the carriage 130 engages the stem 155, causing the retaining portion 145 to deflect via the slot 150. When the retaining portion 145 deflects, the needle assembly 400 is released from the carriage 130 and advances through the seal 210 into the interior of the disposal device 200, as shown in Figures 8 and 9. The seal 210 may return to a non-deflected position after the needle assembly 400 passes through the interface 205.

In an exemplary embodiment, a feedback (e.g., audible, tactile, visual) may be provided to indicate that the needle assembly 400 has been released from the medicament delivery device 300. For example, an exemplary audible feedback may be a sound of the ramped portion 160 snapping over the stem 155. An exemplary tactile feedback may be an increased resistance when the ramped portion 160 engages the stem 155 and/or the ramped portion 160 abutting the base 115.

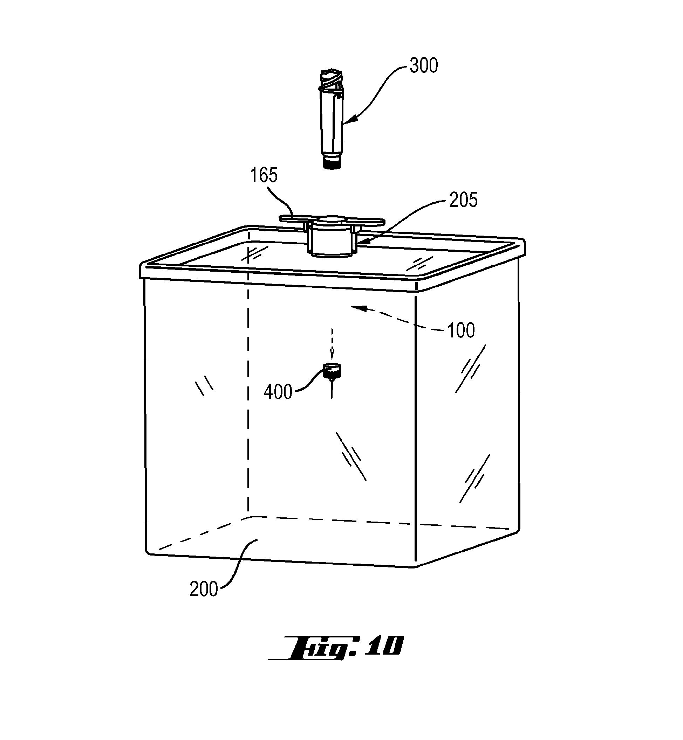

When the carriage 130 is in the second axial position, the spring 135 is compressed. Thus, when axial force being applied to the medicament delivery device 300 is released, the force in the spring 135 may be applied to the medicament delivery device 300, facilitating removal from the needle assembly removal device 100, as shown in Figure 10.

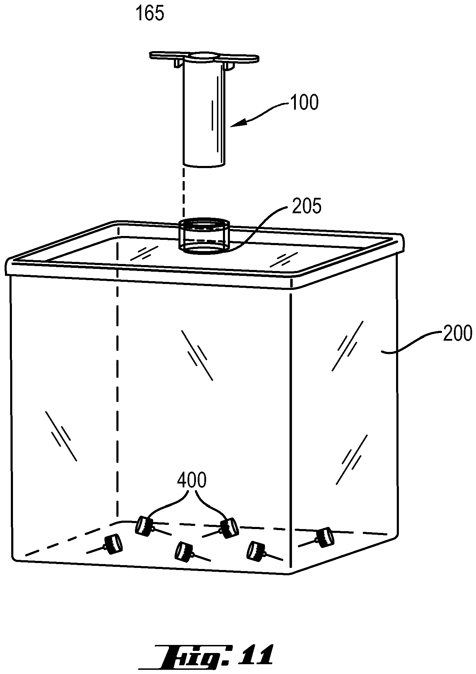

Figure 11 shows an exemplary embodiment of a needle assembly removal device 100 and a disposal device 200 according to the present invention after use. After the needle assembly 400 has been deposited in the disposal device 200 and/or after the disposal device 200 is full, the needle assembly removal device 100 may be detached from the disposal device 200. In an exemplary embodiment in which the needle assembly removal device 100 protrudes at least partially through the interface 205 into the interior of the disposal device 200, when the needle assembly removal device 100 is detached, the seal 210 may return to a non-deflected position.

In an exemplary embodiment, the disposal device 200 may include a cap (not shown) to be disposed over the interface 205 when the needle assembly removal device 100 is detached. The cap may prevent needle assemblies within the disposal device 200 from protruding through the interface 205 during, for example, transport of the disposal device 200, or if the disposal device 200 should tilt or turn-over.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle assembly removal device (100) comprising:
a coupling mechanism adapted to engage a disposal device;
a housing (105); and
a carriage (130) adapted to engage a needle assembly (400),
wherein axial movement of the carriage (130) relative to the housing (105) causes the needle assembly (400) to disengage a medicament delivery device (300).

2. The needle assembly removal device (100) according to claim 1, wherein the coupling mechanism includes at least one of threads, a bayonet mechanism, a friction fit and a snap fit.

3. The needle assembly removal device (100) according to any of the preceding claims, wherein the coupling mechanism is adapted to detachably engage the disposal device.

4. The needle assembly removal device (100) according to any of the preceding claims, wherein the housing (105) includes a proximal opening (110) adapted to receive the medicament delivery device (300) and a base (115) with a distal opening (120).

5. The needle assembly removal device (100) according to any of the preceding claims, wherein the housing (105) includes a track (125) adapted to engage the carriage (130).

6. The needle assembly removal device (100) according to claim 5, wherein the track (125) includes one or more helical grooves.

7. The needle assembly removal device (100) according to claims 1 and 5, wherein the carriage (130) includes at least one peg (126) adapted to engage the one or more helical grooves.

8. The needle assembly removal device (100) according to any of the preceding claims, wherein the carriage (130) is adapted to rotate in a first rotational direction when the carriage (130) moves in a first axial direction relative to the housing (105) and the carriage (13) is adapted to rotate in a second rotational direction when the carriage (130) moves in a second axial direction relative to the housing (105).

9. The needle assembly removal device (100) according to any of the preceding claims, further comprising:
a spring (135) adapted to apply a biasing force to the carriage (130).

10. The needle assembly removal device (100) according to claim 9, wherein the spring (135) biases the carriage (130) in the second axial direction.

11. The needle assembly removal device (100) according to any of the preceding claims, wherein the carriage (130) includes a retaining portion (145) adapted to engage a needle hub (405) of the needle assembly (400) and prevent the needle assembly (400) from rotating relative to the carriage (130).

12. The needle assembly removal device (100) according to claim 11, wherein the carriage (130) includes a slot (150) separating the retaining portion (145), wherein the retaining portion (145) is adapted to deflect radially via the slot (150) to accommodate the needle hub (405).

13. The needle assembly removal device (100) according to claims 4 and 12, wherein the retaining portion (145) includes a ramped portion (160) adapted to engage a stem (155) on the base (115), and wherein the retaining portion (145) deflects radially when the ramped portion (160) engages the stem (155).

14. A disposal device (200) comprising:
an interface (205) adapted to engage the needle assembly removal device (100) according to any of the preceding claims; and
a resilient seal (210) covering the interface (205).

15. The disposal device (200) according to claim 14, further comprising:
a cap adapted to cover the interface (205) and the seal (210).
